# FASCICULE DE BREVET EUROPEEN

(11) **EP 1 273 283 B1**
(45) Date de publication et mention de la délivrance du brevet: **06.10.2004**
(21) Numéro de dépôt: 02291607.6
(22) Date de dépôt: 28.06.2002
(51) Int. Cl.: A61K 7/13

(54) **Précurseurs de colorants phosphatés et leur application pour la teinture des fibres kératiniques**
Phosphatierte Farbstoffvorläufer und ihre Anwendung in der Färbung von Keratinfasern
Phosphated dye precursors and their application for dyeing keratinous fibres

(30) Priorité: 06.07.2001 FR 0109017
(43) Date de publication de la demande: 08.01.2003
(73) Titulaire: L'OREAL, 75008 Paris (FR)
(72) Inventeur: Pereira, Rui, 77140 Montevrain (FR); Martin, Richard, 37210 Rochecorbon (FR)
(74) Mandataire: Casalonga, Axel

(56) Documents cités:
- FR-A- 2 779 951
- GB-A- 1 128 371
- US-A- 3 002 893
- US-A- 5 073 174
- US-A- 5 589 328
- US-A- 5 965 114

## Description

La présente invention est relative à l'utilisation de précurseurs de colorants phosphatés pour la teinture des fibres kératiniques, et en particulier des fibres kératiniques humaines, aux compositions cosmétiques de teinture ainsi qu'au procédé de teinture des fibres kératiniques les mettant en oeuvre.

Il est connu de teindre les fibres kératiniques, et en particulier les cheveux, avec des compositions de teinture contenant des précurseurs de colorants d'oxydation, généralement appelés "bases d'oxydation", en particulier des ortho- ou para-phénylènediamines, des ortho- ou para-aminophénols et des bases hétérocycliques.

Les précurseurs de colorants d'oxydation sont des composés initialement peu ou pas colorés qui développent leur pouvoir tinctorial au sein du cheveu en présence d'un agent oxydant. L'agent oxydant utilisé est généralement le peroxyde d'hydrogène. La formation des composés colorés résulte, soit d'une condensation des "bases d'oxydation" sur elles-mêmes, soit d'une condensation des "bases d'oxydation" sur des composés modificateurs de coloration, ou "coupleurs", qui sont généralement présents dans les compositions tinctoriales utilisées en teinture d'oxydation et qui sont représentés plus particulièrement par des méta-phénylènediamines, des méta-aminophénols et des méta-diphénols et certains composés hétérocycliques.

Ces procédés bien connus dans l'état de la technique ne sont pas totalement satisfaisants et on cherche de ce fait à mettre au point des systèmes de coloration, en particulier des cheveux, à partir de précurseurs de colorants nouveaux.

Il existe aussi des composés hydroxylés tels que l'indoxyle qui forment par oxydation un pigment coloré. Dans le cas de l'indoxyle, il se forme l"indigo, pigment de couleur bleue connu dans son utilisation pour la teinture textile et la coloration des cheveux depuis des millénaires.

Cependant, ce précurseur d'indigo n'est pas stable en solution. Il est donc peu adapté à la préparation de compositions tinctoriales. De plus, l'indigo est un composé insoluble dans l'eau, ce qui entraîne de nombreux problèmes comme la difficulté de pénétration dans les cheveux, la mauvaise uniformité de coloration, l'apparition d'agrégats de pigments, et une instabilité des compositions contenant ce pigment.

Le brevet US N° 5,965,114 de Braun et al. utilise un dérivé de l'indoxyle qui possède un groupe DL, D ou L-aldopyranose ou un groupe DL, D ou L-kétopyranose. Ce dérivé est hydrolysé en indoxyle au moment de l'emploi afin d'obtenir des colorations bleues ou violettes. L'inconvénient lié à ce type de produits est que les enzymes utilisées ne peuvent pas être mises en oeuvre à des pH très alcalins, et les teintures obtenues ne sont pas très couvrantes.

La demanderesse vient de découvrir qu'il est possible d'obtenir sur les fibres kératiniques des teintures bien couvrantes, dans des nuances intenses et variées, en utilisant des précurseurs de colorant portant un substituant phosphate de formule -O-PO(OH)₂, en présence d'une phosphatase acide ou alcaline. Ces précurseurs présentent l'avantage d'être solubles dans l'eau et d'être stables. En présence d'une phosphatase acide ou alcaline, ces précurseurs forment un colorant capable de teindre les fibres kératiniques.

Les colorations obtenues présentent par ailleurs une bonne résistance à la transpiration, à la lumière et aux shampooings.

L'invention a ainsi pour objet l'utilisation pour la teinture des fibres kératiniques des précurseurs de colorants d'oxydation phosphatés, en présence d'au moins une phosphatase acide ou alcaline.

Un autre objet est constitué par les compositions tinctoriales les mettant en oeuvre.

L'invention a également pour objet un procédé de teinture des fibres kératiniques mettant en oeuvre ces colorants.

D'autres objets de l'invention apparaîtront à la lecture de la description et des exemples qui suivent.

Les précurseurs de colorants d'oxydation utilisés pour la teinture des fibre kératiniques et plus particulièrement des fibres kératiniques humaines telles que les cheveux sont des précurseurs de colorants, c'est-à-dire des composés susceptibles de former par oxydation un colorant, ces précurseurs portant au moins un substituant phosphate de formule -O-PO(OH)₂.

Ces composés sont en particulier les composés aromatiques ayant un ou plusieurs cycles condensés, ces cycles pouvant être des carbocycles ou des hétérocycles à 5 ou 6 liaisons contenant un ou plusieurs hétéroatomes choisis parmi l'oxygène et l'azote, et substitués par au moins un groupement phosphate.

Les précurseurs de colorants phosphatés utilisés conformément à l'invention sont choisis de préférence parmi les dérivés benzéniques, naphtaléniques, indoliques ou indoliniques substitués éventuellement par un ou plusieurs atomes d'halogène, par un ou plusieurs groupements carboxyle, alcoxy, aryloxy, nitro, cyano, carbamyle, acylamino, amino, alkylamino, sulfonamido, hydroxyalkyle.

Le ou les atomes d'halogène sont choisis parmi le brome, le chlore, le fluor et l'iode.

Les groupements alcoxy, acylamino, alkylamino et hydroxyalkyle comportent de préférence de un à quatre atomes de carbone.

Les composés pouvant être utilisés en teinture d'oxydation selon l'invention sont notamment : le 3-indoxyl-phosphate, le 4-chloro-3-indoxyl-phosphate, le 6-chloro-3-indoxyl-phosphate, le 5-bromo-6-chloro-3-indoxyl-phosphate, le 5-bromo-4-chloro-3-indoxyl-phosphate, le 6-fluoro-3-indoxyl-phosphate, le 5-iodo-3-indoxyl-phosphate, le N-méthyl-3-indoxyl-phosphate, l'alpha-naphtyl-phosphate de formule 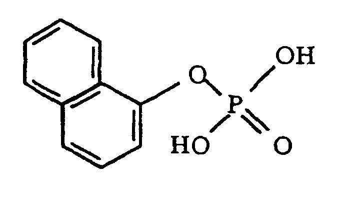 le naphtol AS-TR-phosphate de formule 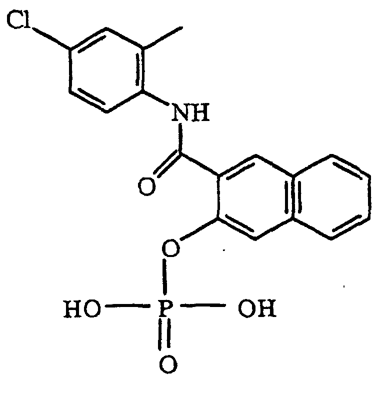 le béta-naphtyl-phosphate de formule 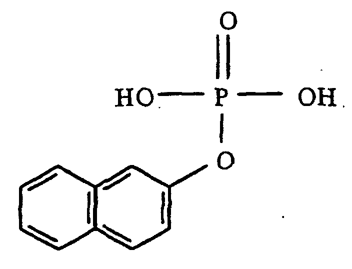 le naphtol AS-BI phosphate de formule 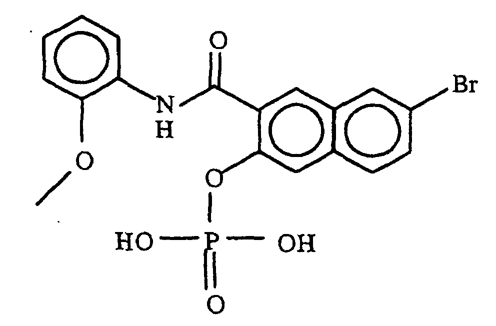 le 4-methylumbelliferyl phosphate de formule 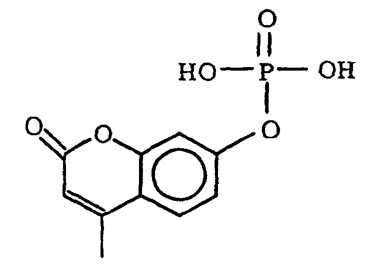 le naphtol AS phosphate de formule 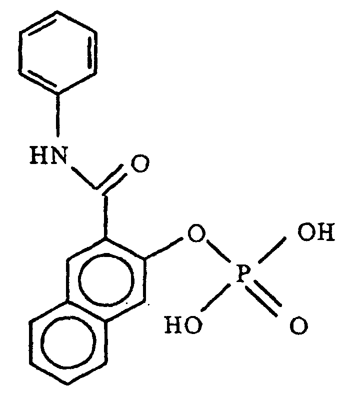 le naphtol AS-OL phosphate de formule 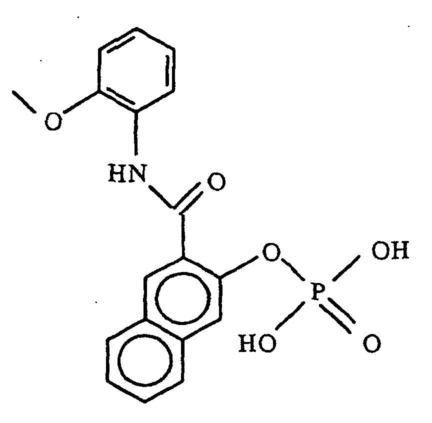 le naphtol AS-KB phosphate de formule 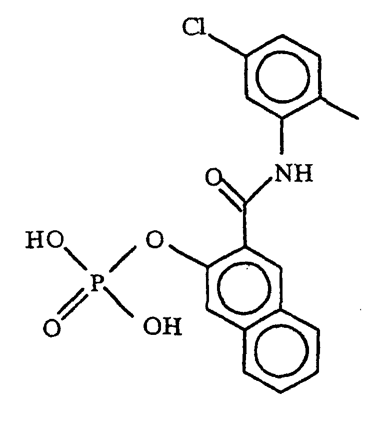 le composé de formule 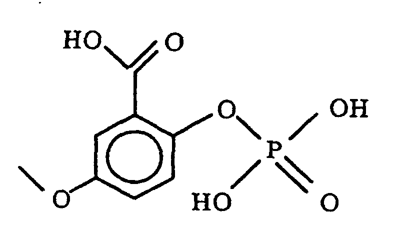 le composé de formule 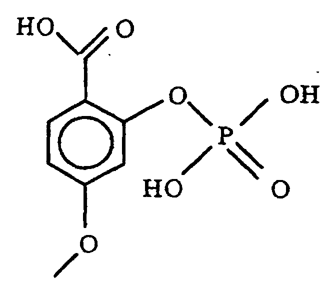 le composé de formule

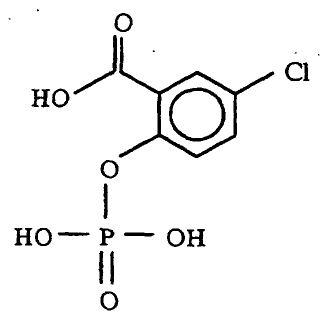

Le précurseur de colorant est de préférence choisi parmi les composés de formule (I) : 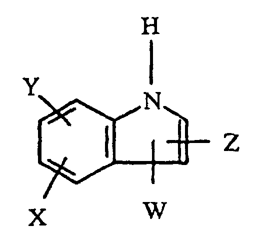 dans laquelle
W, X et Y représentent chacun indépendamment un atome d'hydrogène, un atome d'halogène, un groupe hydroxy, carboxyle ou alcoxy ayant de 1 à 4 atomes de carbone,
Z représente le groupe phosphate -O-PO(OH)₂, ledit groupe Z pouvant être porté par n'importe quel atome de carbone d'un des deux cycles.

Selon un mode de réalisation particulier, le substituant Z est porté par le carbone C-3.

Ces composés sont utilisés en association avec au moins une phosphatase acide ou alcaline.

La phosphatase est une enzyme capable de convertir un monoester phosphorique R-O-PO(OH)₂ en alcool et orthophosphate. Une telle enzyme est également connue sous le nom de phosphomonoesterase acide ou alkaline, glycérophosphatase acide ou alcaline, ou phosphohydrolase acide ou alcaline de monoester d'acide phosphorique.

Les phosphatases alcalines sont répertoriées sous la numérotation E.C.3.1.3.1. et les phosphatases acides sont répertoriées sous la numérotation E.C.3.1.3.2., selon la nomenclature des enzymes décrite dans « Enzyme Nomenclature : Recommendations (1992) of the Nomenclature Comittee of the International Union of Biochemistry and Molecular Biology » publié par Academic press.

Les phosphatases acides ou alcalines utilisées dans la présente invention peuvent notamment être choisies parmi les phosphatases extraites de végétaux, d'animaux, d'insectes ou encore de microorganismes, particulièrement de cellules différenciées ou indifférenciées, obtenues in vivo ou in vitro, avant ou après modifications génétiques.

L'invention a ainsi pour objet l'utilisation pour la teinture des fibres kératiniques, et en particulier des fibres kératiniques humaines telles que les cheveux, d'un précurseur de colorant portant au moins un substituant phosphate de formule -O-PO(OH)₂, avec au moins une phosphatase acide ou alcaline.

Au sens de l'invention, on entend principalement par fibres kératiniques humaines, les cheveux, les poils, les cils et les sourcils, et plus particulièrement les cheveux.

L'invention a également pour objet une composition de teinture des fibres kératiniques, en particulier des fibres kératiniques humaines telles que les cheveux, caractérisée par le fait qu'elle comprend dans un milieu approprié pour la teinture au moins un des précurseurs de colorant définis plus haut et au moins une phosphatase acide ou alcaline.

Le pH de la composition peut être compris entre 7 et 11 et préférentiellement entre 8 et 10.

Le composé de formule (I) est de préférence présent dans la composition dans des proportions comprises entre 0,2 et 10%, et encore de préférence entre 0,5 et 2% du poids total de la composition.

La phosphatase alcaline ou acide peut être présente dans la composition dans une concentration comprise entre 50 et 2000 unités d'activité pour 100 g de composition, de préférence comprise entre 200 et 500 unités d'activité pour 100 g de composition, l'activité enzymatique étant définie comme la quantité de substrat nécessaire pour convertir une µmole de substrat par minute dans 50 µmol de diéthanolamine à un pH de 9,5 ajusté par NaOH ou HCl, à une température de 37°C.

La composition peut également contenir un agent oxydant. Cet agent oxydant peut être choisi parmi les agents oxydants classiquement utilisés pour la teinture d'oxydation des fibres kératiniques, tels que le peroxyde d'hydrogène, le peroxyde d'urée, les bromates de métaux alcalins, les persels tels que les perborates et les persulfates, les enzymes telles que les oxydo-réductases à deux électrons, les oxydo-réductases à quatre électrons tels que les laccases, produisant in situ des peroxydes d'hydrogène en présence de l'oxygène de l'air, ces systèmes contenant par exemple des glucoses oxydases, des choline oxydases, des galactose oxydases, des uricases, et les peroxydases comme la peroxydase de raifort, le cytochrome C, la chloroperoxydase et la microperoxydase.

La composition tinctoriale peut contenir en outre des bases d'oxydation différentes des composés de formule (I) et choisis par exemple parmi les paraphénylènediamines, les bis-phénylalkylènediamines, les para-aminophénols, les ortho-aminophénols les bases hétérocycliques, et/ou des coupleurs choisis parmi les métaphénylènediamines, les méta-aminophénols, les métadiphénols, et les coupleurs hétérocyclique.

Parmi les paraphénylènediamines, on peut plus particulièrement citer à titre d'exemple, la paraphénylènediamine, la paratoluylène diamine, la 2-chloroparaphénylènediamine, la 2,3-diméthyl paraphénylènediamine, la 2,6 diméthyl paraphénylènediamine, la 2,6 diéthyl paraphénylènediamine, la 2,5 diméthyl paraphénylènediamine, la N,N diméthyl paraphénylènediamine, , la N,N-diéthyl paraphénylènediamine, , la N,N-dipropyl paraphénylènediamine la 4-amino N,N-diéthyl 3-méthyl aniline, la N,N-bis-(β-hydroxyethyl)paraphénylènediamine, la 4-N,N-bis-(β-hydroxyethyl)amino 2-méthyl aniline, la 4-N,N-bis-(β-hydroxyethyl)amino 2-chloro aniline, la 2-β-hydroxyethyl paraphénylènediamine, la 2-fluoro paraphénylènediamine, la 2-isopropyl paraphénylènediamine, la N-(β-hydroxypropyl)paraphénylènediamine, la 2-hydroxyméthyl paraphénylènediamine, la N,N-diméthyl 3-méthyl paraphénylènediamine, la N,N-(éthyl,β-hydroxyethyl)paraphénylène-diamine, la N-(β-γ-dihydroxypropyl)paraphénylènediamine, la N-(4'-aminophényl) paraphénylènediamine, la N-phénylparaphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine (PPD), la paratoluylènediamine, la 2-isopropyl paraphénylènediamine, la 2- β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl)paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'bis-(β-hydroxyéthyl) N,N'-bis-(4'-aminophényl) 1,3-diamino propanol, la N,N'-bis-(β-hydroxyéthyl)N,N'-bis-(4'-aminophényl)éthylènediamine, laN'N'-bis-(4-aminophényl)tétraméthylènediamine, la N,N'bis-(β-hydroxyéthyl) N,N'bis-(4-aminophényl) tétraméthylènediamine, la N,N'-bis-(4-méthylaminophényl) tétraméthylènediamine, la N,N'bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à tire d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl-aminométhyl) phénol, le 4-amino 2-fluoro phénol, et leurs sels d'addition avec un acide.

Parmi les ortho-aminophénols, on peut plus particulièrement citer à titre d'exemple le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl)pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-( β-hydroxyéthyl) 3-méthyl pyrazole, le 4,5-diamino 1-éthyl 3-méthyl pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthylpyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12% en poids environ du poids total de la composition tinctoriale, et encore plus préférentiellement de 0,005 à 6% en poids environ de ce poids.

La composition tinctoriale conforme à l'invention peut également renfermer un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy)benzène, le 2-amino 4-(β-hydroxyéthylamino) 1-méthoxy benzène, le 1,3-diamino benzène, le 1,3-bis-(2,4-diaminophénoxy)propane, le 4-hydroxy N-méthyl indole, la 2,6-dihydroxy 4-méthyl pyridine, le 1-H 3-méthyl pyrazole 5-one, le 1-phényl 3-méhyl pyrazole 5-one, et leurs sels d'addition avec un acide.

Lorsqu'ils sont présents ces coupleurs additionnels représentent de préférence de 0,0001 à 10% en poids environ du poids total de la composition tinctoriale et encore plus préférentiellement de 0,005 à 5% en poids environ de ce poids.

D'une manière générale, les sels d'addition avec un acide utilisable dans le cadre des compositions tinctoriales de l'invention (bases d'oxydation et coupleurs) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40% en poids environ par rapport au poids total de la composition tinctoriale, et encore plus préférentiellement entre 5 et 30% en poids environ.

Le pH de la composition tinctoriale conforme à l'invention peut être ajusté à la valeur désirée au moyen d'agents acidifiants ou alcalinisants habituellement utilisés en teinture des fibres kératiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 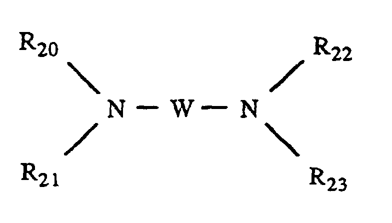 dans laquelle W est un reste propylène substitué ou non substitué par un groupement hydroxyle ou un radical alkyle en C₁-C₆ ; R₂₀, R₂₁, R₂₂ et R₂₃, identiques ou différents, représentent un atome d'hydrogène, un radical alkyle en C₁-C₆ ou hydroxyalkyle en C₁-C₆.

On peut aussi utiliser des milieux tampons, par exemple à partir de diéthanolamine, de borate, de 2-amino-2-méthyl-1-propanol, glycine, triméthylamine, pyrophosphate, méthylamine, carbonate.

La composition tinctoriale peut également contenir des colorants directs tels que les colorants azoïques, anthraquinoniques et les dérivés nitrés de la série benzénique ou des précurseurs de mélanine.

Les colorants nitrés benzéniques sont par exemple les nitrophénylènediamines, les nitrodiphénylamines, les nitroanilines, les éthers de phénol nitrés ou les nitrophénols, les nitropyridines, les colorants anthraquinoniques, mono- ou di-azoïques, triarylméthaniques, aziniques, acridiniques et xanthéniques, ou encore les colorants métallifères. La proportion de tous ces colorants directs d'addition peut varier entre 0,05 et 10 % en poids par rapport au poids total de la composition tinctoriale.

Ladite composition tinctoriale peut contenir en outre tout autre adjuvant utilisé habituellement en teinture des matières kératiniques, et par exemple, des agents tensioactifs connus de l'art antérieur et de type anionique, cationique, non-ionique, amphotère, zwittérionique ou leurs mélanges, des agents épaississants, des agents anti-oxydants, des parfums, des agents séquestrants, des agents dispersants, des agents de conditionnement, des agents conservateurs, des agents opacifiants, etc...

La composition appliquée sur les cheveux peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Bien entendu, l'homme de l'art veillera à choisir le ou les éventuels composés complémentaires mentionnés ci-dessus, de telle manière que les propriétés avantageuses liées intrinsèquement à la composition tinctoriale selon l'invention ne soient pas, ou substantiellement pas, altérées par la ou les adjonctions envisagées.

La présente invention a également pour objet un procédé de teinture des fibres kératiniques, et en particulier des fibres kératiniques humaines telles que les cheveux, consistant à appliquer sur les matières kératiniques une des compositions définies précédemment.

Un des objets de la présente invention est également un procédé de teinture des fibres kératiniques, en particulier des fibres kératiniques humaines telles que les cheveux, qui consiste à appliquer sur lesdites fibres au moins un composant (A) constitué d'une composition renfermant dans un milieu approprié pour la teinture, au moins un des précurseurs de colorants phosphatés définis plus haut, et au moins un composant (B) constitué d'une composition contenant dans un milieu approprié pour la teinture, au moins une phosphatase alcaline ou acide, le composant B étant mélangé extemporanément avec le composant A tout juste avant emploi ou appliqué de façon séquentielle avant, ou après, ou simultanément avec le composant A. Un agent oxydant peut être intégré dans le composant (B).

Selon un procédé de l'invention, les composants (A) et (B) sont mélangés juste avant emploi. Le mélange obtenu est alors appliqué immédiatement sur les fibres kératiniques. Puis on laisse agir pendant 1 à 60 minutes, de préférence pendant 1 à 30 minutes. Les fibres kératiniques sont ensuite rincées, lavées au shampooing, rincées à nouveau, puis séchées.

Il est également possible d'appliquer successivement sur les fibres kératiniques les composants (A) et (B), l'ordre d'application étant indifférent, de laisser agir pendant 1 à 60 minutes, de préférence pendant 1 à 30 minutes, de procéder éventuellement au rinçage à l'eau entre chaque application, puis de rincer, laver au shampooing, rincer à nouveau, puis sécher lesdites fibres kératiniques.

La présente invention a également pour objet un agent de teinture des fibres kératiniques, et en particulier des fibres kératiniques humaines telles que les cheveux, comportant les composants (A) et (B) définis précédemment, sous forme séparée, les composants (A) et (B) étant destinés, soit à être mélangés juste avant emploi, soit à être appliqués de façon successive sur les fibres à traiter.

Un agent oxydant peut être intégré dans le composant (B).

La présente invention a également pour objet un dispositif à plusieurs compartiments ou "kit de teinture", qui comporte au moins deux compartiments dont le premier renferme le composant (A) et le second renferme le composant (B).

Un agent oxydant peut être intégré dans le composant (B).

Le dispositif à plusieurs compartiments ou "kit de teinture" définis ci-dessus peut comporter trois compartiments, le premier contenant le composant (A), le deuxième contenant le composant (B), l'un au moins des composants (A) et (B) se présentant sous forme de composition anhydre, le troisième contenant un milieu aqueux approprié pour la teinture, ledit milieu étant destiné à être mélangé avant emploi avec l'un au moins des composants (A) et (B).

Les exemples suivants, nullement limitatifs, sont destinés à illustrer l'invention.

### EXEMPLES

La phosphatase utilisée dans les exemples suivants est la phosphatase Escherichia coli K12, fournie par la société sigma.

### Exemple 1

On prépare juste avant emploi la composition de teinture suivante:

| | |
|---|---|
| 3-indoxyl-phosphate | 0,5 g |
| Phosphatase alcaline | 400 unités |
| Tampon de diéthanolamine dans l'eau 0,1M à pH 9,6 | 94,5 g |

On applique la composition ci-dessus sur des mèches de cheveux gris naturels à 90% de blancs et on laisse poser pendant 30 minutes. Après rinçage à l'eau courante et séchage, les cheveux sont teints dans une nuance bleu foncée indigo.

### Exemple 2

On prépare juste avant emploi la composition de teinture suivante:

| | |
|---|---|
| 5-bromo-6-chloro-3-indoxyl-phosphate | 0,5 g |
| Phosphatase alcaline | 400 unités |
| Tampon de diéthanolamine dans l'eau 0,1M à pH 9,6 | 94,5 g |

On applique la composition ci-dessus sur des mèches de cheveux gris naturels à 90% de blancs et on laisse poser pendant 30 minutes. Après rinçage à l'eau courante et séchage, les cheveux sont teints dans une nuance magenta.

### Exemple 3

On prépare juste avant emploi la composition de teinture suivante:

| | |
|---|---|
| 5-bromo-4-chloro-3-indoxyl-phosphate | 0,5 g |
| Phosphatase alcaline | 400 unités |
| Tampon de diéthanolamine dans l'eau 0,1M à pH9,6 | 94,5 g |

On applique la composition ci-dessus sur des mèches de cheveux gris naturels à 90% de blancs et on laisse poser pendant 30 minutes. Après rinçage à l'eau courante et séchage, les cheveux sont teints dans une nuance bleue.

### Exemple 4

On prépare des compositions de teinture en procédant aux étapes suivantes :

### Etape 1

Les composés suivants sont aioutés dans les quantités indiquées :

| **Composé** | **Concentration finale** |
|---|---|
| Tampon diéthanolamine | 0,106 M |
| Composé phosphaté | 1 mM |
| Base ou coupleur | 1 mM |
| Phosphatase alcaline | 2500U/100g |

On applique cette composition sur des mèches de cheveux blancs (90% de cheveux blancs) naturels.

On procède ensuite à une incubation des mèches pendant 30 minutes à 37°C.

### Etape 2

On applique ensuite de l'eau oxygénée à 4 volumes (1,2% en poids). On laisse poser pendant 15 minutes, puis on lave, on rince et on sèche.

Les résultats obtenus avec trois composés phosphatés sont indiqués dans le tableau ci-dessous.

| **Composé phosphaté** | **Base ou coupleur** | **Couleur** |
|---|---|---|
| Alpha-naphtyl-phosphate | 4-amino-phénol | saumon |
| | 5-méthylpyrazolo[1,5a]pyrimidine-3,7diamine | Rose foncé |
| | PPD | violet |
| | 4,5-diamino-1-ethyl-3-methylpyrazole | violet |
| | 5-amino-2-méthylphénol | violet |
| Naphthol AS-TR-phosphate | 5-méthylpyrazolo[1,5a]pyrimidine-3,7diamine | Bleu cyan |
| Beta-naphthyl-phosphate | 5-méthylpyrazolo[1,5a]pyrimidine-3,7diamine | violet |

## Revendications

1. Utilisation pour la teinture des fibres kératiniques, en particulier des fibres kératiniques humaines telles que les cheveux, d'un précurseur de colorant portant au moins un substituant phosphate de formule -O-PO(OH)₂, en présence d'au moins une phosphatase acide ou alcaline.

2. Utilisation selon la revendication 1, **caractérisée en ce que** le précurseur de colorant est un composé aromatique ayant un ou plusieurs cycles condensés, ces cycles pouvant être des carbocycles ou des hétérocycles à 5 ou 6 liaisons contenant un ou plusieurs hétéroatomes choisis parmi l'oxygène et l'azote, et substitués par au moins un groupement phosphate.

3. Utilisation selon la revendication 1 ou 2, **caractérisée par le fait que** le précurseur de colorant est choisi parmi les composés de formule (I) : 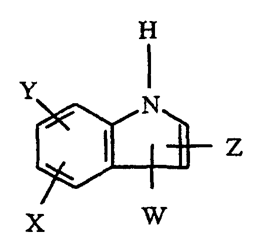 dans laquelle
W, X et Y représentent chacun indépendamment un atome d'hydrogène, un atome d'halogène, un groupe hydroxy, carboxyle ou alcoxy ayant de 1 à 4 atomes de carbone,
Z représente le groupe phosphate -O-PO(OH)₂, ledit groupe Z pouvant être porté par n'importe quel atome de carbone d'un des deux cycles.

4. Utilisation selon la revendication 3, **caractérisée par le fait que** le substituant Z est porté par le carbone C-3.

5. Utilisation selon la revendication 3 ou 4, **caractérisée par le fait que** le composé de formule (I) est choisi parmi le 3-indoxyl phosphate, le 4-chloro-3-indoxyl phosphate, le 6-chloro-3-indoxyl-phosphate, le 5-bromo-6-chloro-3-indoxyl-phosphate, le 5-bromo-4-chloro-3-indoxyl-phosphate, le 6-fluoro-3-indoxyl-phosphate, le 5-iodo-3-indoxyl-phosphate, et le N-méthyl-3-indoxyl-phosphate.

6. Utilisation selon l'une quelconque des revendications précédentes, **caractérisée par le fait que** la phosphatase acide ou alcaline est extraite de plantes, animaux, insectes, microorganismes.

7. Composition de teinture des fibres kératiniques, en particulier des fibres kératiniques humaines telles que les cheveux, **caractérisée par le fait qu'**elle comprend dans un milieu approprié pour la teinture au moins un précurseur de colorant défini dans l'une quelconque des revendications 1 à 5, et au moins une phosphatase acide ou alcaline.

8. Composition de teinture selon la revendication 7, **caractérisée par le fait qu'**elle a un pH compris entre 7 et 11 et préférentiellement entre 8 et 10.

9. Composition selon la revendication 7 ou 8, **caractérisée par le fait que** le composé de formule (I) est présent dans des proportions comprises entre 0,2 et 10 %, et de préférence de 0,5 à 2% du poids total de la composition.

10. Composition selon l'une quelconque des revendications 7 à 9, **caractérisée par le fait que** la phosphatase alcaline ou acide est présente dans une concentration comprise entre 50 et 2000 unités d'activité pour 100 g de composition, de préférence comprise entre 200 et 500 unités d'activité pour 100 g de composition.

11. Composition selon l'une quelconque des revendications 7 à 10, **caractérisée par le fait qu'**elle contient en outre un agent oxydant.

12. Composition selon l'une quelconque des revendications 7 à 11, **caractérisée par le fait qu'**elle contient en outre des bases d'oxydation différentes des composés de formule (I) et choisis parmi les paraphénylènediamines, les bis-phénylalkylènediamines, les para-aminophénols, les ortho-aminophénols, les bases hétérocycliques et/ou des coupleurs choisis parmi les métaphénylènediamines, les méta-aminophénols, les méta-diphénols, et les coupleurs hétérocycliques.

13. Composition selon l'une quelconque des revendications 7 à 12, **caractérisée par le fait qu'**elle contient en outre des colorants directs choisis parmi les colorants azoïques, anthraquinoniques, les dérivés nitrés de la série benzénique, et les précurseurs de la mélanine

14. Composition selon l'une quelconque des revendications 7 à 13, **caractérisée par le fait que** le milieu approprié pour la teinture est un milieu contenant de l'eau ou un mélange d'eau et d'au moins un solvant organique choisi parmi les alcools, les glycols et les éthers de glycol, les solvants étant présents dans des proportions comprises entre 1 et 25% du poids total de la composition.

15. Procédé de teinture des fibres kératiniques, en particulier des fibres kératiniques humaines telles que les cheveux, **caractérisé par le fait qu'**il consiste à appliquer sur lesdites fibres la composition selon l'une quelconque des revendications 7 à 14.

16. Procédé de teinture des fibres kératiniques, en particulier des fibres kératiniques humaines telles que les cheveux, **caractérisé par le fait qu'**il consiste à appliquer sur lesdites fibres au moins un composant (A) constitué d'une composition renfermant dans un milieu approprié pour la teinture, au moins un des précurseur de colorants phosphatés définis dans l'une quelconque des revendications 1 à 5, et au moins un composant (B) constitué d'une composition contenant, dans un milieu approprié pour la teinture, au moins une phosphatase alcaline ou acide, le composant B étant mélangé extemporanément avec le composant A tout juste avant emploi, ou appliqué de façon séquentielle avant, ou après, ou simultanément avec le composant A.

17. Procédé selon la revendication 16, **caractérisé par le fait qu'**il comprend les étapes consistant à mélanger les composants (A) et (B) juste avant emploi, à appliquer immédiatement le mélange obtenu sur les fibres kératiniques, à laisser agir pendant 1 à 60 minutes, de préférence pendant 1 à 30 minutes, à rincer, laver au shampooing, rincer à nouveau, puis à sécher lesdites fibres kératiniques.

18. Procédé selon la revendication 16, **caractérisé par le fait qu'**il comprend les étapes consistant à appliquer successivement sur les fibres kératiniques les composants (A) et (B), l'ordre d'application étant indifférent, à laisser agir pendant 1 à 60 minutes, de préférence pendant 1 à 30 minutes, à procéder éventuellement au rinçage à l'eau entre chaque application, à rincer, laver au shampooing, rincer à nouveau, puis sécher lesdites fibres kératiniques.

19. Agent de teinture des fibres kératiniques, et en particulier des fibres kératiniques humaines telles que les cheveux, **caractérisé par le fait qu'**il comporte les composants (A) et (B) tels que définis dans la revendication 16, sous forme séparée, les composants (A) et (B) étant destinés, soit à être mélangés juste avant emploi, soit à être appliqués de façon successive sur les fibres à traiter.

20. Dispositif à plusieurs compartiments ou "kit de teinture", **caractérisé par le fait qu'**il comporte au moins deux compartiments dont le premier renferme le composant (A) tel que défini dans la revendication 16, et le second renferme le composant (B) tel que défini dans la revendication 16.

21. Dispositif selon la revendication 20, **caractérisé par le fait qu'**il comporte trois compartiments, le premier contenant le composant (A), le deuxième contenant le composant (B), l'un au moins des composants (A) et (B) se présentant sous forme de composition anhydre, le troisième compartiment contenant un milieu aqueux approprié pour la teinture, ledit milieu étant destiné à être mélangé avant emploi avec l'un au moins des composants (A) et (B).

## Claims

1. Use for dyeing keratin fibres, in particular human keratin fibres such as the hair, of a dye precursor bearing at least one phosphate substituent of formula -O-PO(OH)₂, in the presence of at least one acid or alkaline phosphatase.

2. Use according to Claim 1, **characterized in that** the dye precursor is an aromatic compound containing one or more fused rings, these rings possibly being 5-or 6-membered carbocyles or heterocycles containing one or more hetero atoms chosen from oxygen and nitrogen, and substituted with at least one phosphate group.

3. Use according to Claim 1 or 2, **characterized in that** the dye precursor is chosen from the compounds of formula (I) : 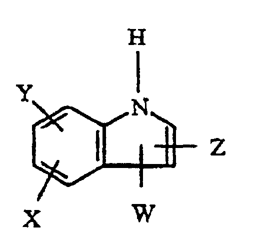 in which
W, X and Y each independently represent a hydrogen atom, a halogen atom or a hydroxyl, carboxyl or alkoxy group containing from 1 to 4 carbon atoms,
Z represents a phosphate group -O-PO(OH)₂, the said group Z possibly being borne by any carbon atom of either of the two rings.

4. Use according to Claim 3, **characterized in that** the substituent Z is borne by the carbon C-3.

5. Use according to Claim 3 or 4, **characterized in that** the compound of formula (I) is chosen from 3-indoxyl phosphate, 4-chloro-3-indoxyl phosphate, 6-chloro-3-indoxyl phosphate, 5-bromo-6-chloro-3-indoxyl phosphate, 5-bromo-4-chloro-3-indoxyl phosphate, 6-fluoro-3-indoxyl phosphate, 5-iodo-3-indoxyl phosphate and N-methyl-3-indoxyl phosphate.

6. Use according to any one of the preceding claims, **characterized in that** the acid or alkaline phosphatase is extracted from plants, animals, insects or microorganisms.

7. Composition for dyeing keratin fibres, in particular human keratin fibres such as the hair, **characterized in that** it comprises, in a medium that is suitable for dyeing, at least one dye precursor defined in any one of Claims 1 to 5, and at least one acid or alkaline phosphatase.

8. Dye composition according to Claim 7, **characterized in that** it has a pH of between 7 and 11 and preferably between 8 and 10.

9. Composition according to Claim 7 or 8, **characterized in that** the compound of formula (I) is present in proportions of between 0.2% and 10% and preferably from 0.5% to 2% relative to the total weight of the composition.

10. Composition according to any one of Claims 7 to 9, **characterized in that** the alkaline or acid phosphatase is present in a concentration of between 50 and 2000 activity units per 100 g of composition and preferably between 200 and 500 activity units per 100 g of composition.

11. Composition according to any one of Claims 7 to 10, **characterized in that** it also contains an oxidizing agent.

12. Composition according to any one of Claims 7 to 11, **characterized in that** it also contains oxidation bases other than the compounds of formula (I) and chosen from para-phenylenediamines, bis(phenyl) alkylenediamines, para-aminophenols, ortho-aminophenols, heterocyclic bases and/or couplers chosen from meta-phenylenediamines, meta-aminophenols, meta-diphenols and heterocyclic couplers.

13. Composition according to any one of Claims 7 to 12, **characterized in that** it also contains direct dyes chosen from azo dyes, anthraquinone dyes, nitrobenzene derivatives and melanin precursors.

14. Composition according to any one of Claims 7 to 13, **characterized in that** the medium that is suitable for dyeing is a medium containing water or a mixture of water and at least one organic solvent chosen from alcohols, glycols and glycol ethers, the solvents being present in proportions of between 1% and 25% relative to the total weight of the composition.

15. Process for dyeing keratin fibres, in particular human keratin fibres such as the hair, **characterized in that** it consists in applying to the said fibres the composition according to any one of Claims 7 to 14.

16. Process for dyeing keratin fibres, in particular human keratin fibres such as the hair, **characterized in that** it consists in applying to the said fibres at least one component (A) consisting of a composition containing, in a medium that is suitable for dyeing, at least one of the phosphate dye precursors defined in any one of claims 1 to 5, and at least one component (B) consisting of a composition containing, in a medium that is suitable for dyeing, at least one alkaline or acid phosphatase, component (B) being extemporaneously mixed with component (A) just before use or applied sequentially before, after or simultaneously with component (A).

17. Process according to Claim 16, **characterized in that** it comprises the steps consisting in mixing components (A) and (B) just before use, immediately applying the mixture obtained to the keratin fibres, leaving it to act for 1 to 60 minutes and preferably for 1 to 30 minutes, rinsing, washing with shampoo, rinsing again and then drying the said keratin fibres.

18. Process according to Claim 16, **characterized in that** it comprises the steps consisting in successively applying to the keratin fibres components (A) and (B), the order of application being irrelevant, leaving them to act for 1 to 60 minutes and preferably for 1 to 30 minutes, optionally rinsing with water between each application, rinsing, washing with shampoo, rinsing again and then drying the said keratin fibres.

19. Agent for dyeing keratin fibres, and in particular human keratin fibres such as the hair, **characterized in that** it comprises components (A) and (B) as defined in Claim 16, in separate form, components (A) and (B) being intended either to be mixed just before use or to be applied successively to the fibres to be treated.

20. Multi-compartment device or "dyeing kit", **characterized in that** it comprises at least two compartments, the first of which contains component (A) as defined in Claim 16, and the second of which contains component (B) as defined in Claim 16.

21. Device according to Claim 20, **characterized in that** it comprises three compartments, the first containing component (A), the second containing component (B), at least one of the components (A) and (B) being in the form of an anhydrous composition, the third compartment containing an aqueous medium that is suitable for dyeing, the said medium being intended to be mixed before use with at least one of the components (A) and (B).

## Patentansprüche

1. Verwendung eines Farbstoffvorproduktes eines Oxidationsfarbstoffes, das mindestens einen Phosphatsubstituenten der Formel -O-PO(OH)₂ aufweist, zum Färben von Keratinfasern und insbesondere menschlichen Keratinfasern, wie dem Haar, in Gegenwart mindestens einer sauren oder alkalischen Phosphatase.

2. Verwendung nach Anspruch 1, **dadurch gekennzeichnet, dass** das Farbstoffvorprodukt eines Oxidationsfarbstoffes eine aromatische Verbindung ist, die einen oder mehrere kondensierte Ringe aufweist, wobei es sich bei den Ringen um Carbocyclen oder Heterocyclen mit 5 oder 6 Bindungen handeln kann, die ein oder mehrere Heteroatome enthalten, die unter Sauerstoff und Stickstoff ausgewählt sind, und die mit mindestens einer Phosphatgruppe substituiert sind.

3. Verwendung nach Anspruch 1 oder 2, **dadurch gekennzeichnet, dass** das Farbstoffvorprodukt eines Oxidationsfarbstoffes unter den Verbindungen der Formel (I) ausgewählt ist, 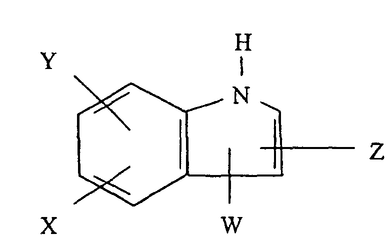 worin bedeuten:
W, X und Y unabhängig voneinander ein Wasserstoffatom, ein Halogenatom, eine Hydroxygruppe, eine Carboxygruppe oder eine Alkoxygruppe mit 1 bis 4 Kohlenstoffatomen,
Z die Phospatgruppe -O-PO(OH)₂, wobei die Gruppe Z an einem der beiden Ringe an einem beliebigen Kohlenstoffatom gebunden sein kann.

4. Verwendung nach Anspruch 3, **dadurch gekennzeichnet, dass** sich der Substituent Z an dem Kohlenstoff C-3 befindet.

5. Verwendung nach Anspruch 3 oder 4, **dadurch gekennzeichnet, dass** die Verbindung der Formel (I) unter 3-Indoxylphosphat, 4-Chlor-3-indoxylphosphat, 6-Chlor-3-indoxylphosphat, 5-Brom-6-chlor-3-indoxylphosphat, 5-Brom-4-chlor-3-indoxylphosphat, 6-Fluor-3-indoxylphosphat, S-Iod-3-indoxylphosphat und N-Methyl-3-indoxylphosphat ausgewählt ist.

6. Verwendung nach einem der vorhergehenden Ansprüche, **dadurch gekennzeichnet, dass** die saure oder alkalische Phosphatase aus Pflanzen, Tieren, Insekten oder Mikroorganismen gewonnen wird.

7. Zusammensetzung zum Färben von Keratinfasern und insbesondere menschlichen Keratinfasern, wie dem Haar, **dadurch gekennzeichnet, dass** sie in einem zum Färben geeigneten Medium mindestens ein Farbstoffvorprodukt eines Oxidationsfarbstoffes nach einem der Ansprüche 1 bis 5 und mindestens eine saure oder alkalische Phosphatase enthält.

8. Zusammensetzung zum Färben nach Anspruch 7, **dadurch gekennzeichnet, dass** sie einen pH-Wert im Bereich von 7 bis 11 und vorzugsweise 8 bis 10 aufweist.

9. Zusammensetzung nach Anspruch 7 oder 8, **dadurch gekennzeichnet, dass** die Verbindung der Formel (I) in Mengenanteilen von 0,2 bis 10 % und vorzugsweise 0,5 bis 2 % des Gesamtgewichts der Zusammensetzung vorliegt.

10. Zusammensetzung nach einem der Ansprüche 7 bis 9, **dadurch gekennzeichnet, dass** die alkalische oder saure Phosphatase in einer Konzentration von 50 bis 2000 Aktivitätseinheiten auf 100 g Zusammensetzung und vorzugsweise 200 bis 500 Aktivitätseinheiten auf 100 g Zusammensetzung enthalten ist.

11. Zusammensetzung nach einem der Ansprüche 7 bis 10, **dadurch gekennzeichnet, dass** sie ferner ein Oxidationsmittel enthält.

12. Zusammensetzung nach einem der Ansprüche 7 bis 11, **dadurch gekennzeichnet, dass** sie ferner Oxidationsbasen, die von den Verbindungen der Formel (I) verschieden sind und die unter den p-Phenylendiaminen, Bisphenylalkylendiaminen, p-Aminophenolen, o-Aminophenolen und heterocyclischen Basen ausgewählt sind, und/oder Kuppler enthält, die unter den m-Phenylendiaminen, m-Aminophenolen, m-Dihydroxybenzolen und heterocyclischen Kupplern ausgewählt sind.

13. Zusammensetzung nach einem der Ansprüche 7 bis 12, **dadurch gekennzeichnet, dass** sie ferner Direktfarbstoffe enthält, die unter den Azofarbstoffen, Anthrachinon-Farbstoffen, nitrierten Derivaten aus der Benzolgruppe und Melaninprecursoren ausgewählt sind.

14. Zusammensetzung nach einem der Ansprüche 7 bis 13, **dadurch gekennzeichnet, dass** das zum Färben geeignete Medium ein Medium ist, das Wasser oder ein Gemisch von Wasser und mindestens einem organischen Lösungsmittel enthält, das unter den Alkoholen, Glykolen und Glykolethern ausgewählt ist, wobei die Lösungsmittel in Mengenanteilen von 1 bis 25 % des Gesamtgewichts der Zusammensetzung enthalten sind.

15. Verfahren zum Färben von Keratinfasern und insbesondere menschlichen Keratinfasern, wie dem Haar, **dadurch gekennzeichnet, dass** es darin besteht, auf die Fasern die Zusammensetzung nach einem der Ansprüche 7 bis 14 aufzutragen.

16. Verfahren zum Färben von Keratinfasern, insbesondere menschlichen Keratinfasern, wie dem Haar, **dadurch gekennzeichnet, dass** es darin besteht, auf die Fasern mindestens eine Komponente (A), die aus einer Zusammensetzung besteht, die in einem zum Färben geeigneten Medium mindestens ein phosphatiertes Farbstoffvorprodukt eines Oxidationsfarbstoffes nach einem der Ansprüche 1 bis 5 enthält, und mindestens eine Komponente (B) aufzutragen, die aus einer Zusammensetzung besteht, die in einem zum Färben geeigneten Medium mindestens eine alkalische oder saure Phosphatase enthält, wobei die Komponente (B) kurz vor der Anwendung bedarfsgemäß mit der Komponente (A) vermischt wird oder getrennt vorher oder nachher oder gleichzeitig mit der Komponente (A) aufgebracht wird.

17. Verfahren nach Anspruch 16, **dadurch gekennzeichnet, dass** es die folgenden Schritte umfasst: Mischen der Komponenten (A) und (B) kurz vor der Anwendung, sofortiges Aufbringen des erhaltenen Gemisches auf die Keratinfasern, Einwirkenlassen während 1 bis 60 min und vorzugsweise 1 bis 30 min, Spülen, Waschen mit Haarwaschmittel, nochmaliges Spülen und anschließend Trocknen der Keratinfasern.

18. Verfahren nach Anspruch 16, **dadurch gekennzeichnet, dass** es die folgenden Schritte umfasst: Aufbringen der Komponenten (A) und (B) nacheinander, wobei die Reihenfolge des Auftragens unerheblich ist, Einwirkenlassen während 1 bis 60 min und vorzugsweise 1 bis 30 min, gegebenenfalls Spülen mit Wasser zwischen den beiden Anwendungen, Spülen, Waschen mit Haarwaschmittel, nochmaliges Spülen und dann Trocknen der Keratinfasern.

19. Mittel zum Färben von Keratinfasern und insbesondere menschlichen Keratinfasern, wie dem Haar, **dadurch gekennzeichnet, dass** es die Komponenten (A) und (B) nach Anspruch 16 in getrennter Form umfasst, wobei die Komponenten (A) und (B) entweder kurz vor der Anwendung vermischt werden sollen oder nacheinander auf die zu behandelnden Fasern aufgetragen werden sollen.

20. Vorrichtung mit mehreren Abteilungen oder "Kit zum Färben", **dadurch gekennzeichnet, dass** sie mindestens zwei Abteilungen aufweist, wobei eine Abteilung die Komponente (A) nach Anspruch 16 und die zweite Abteilung die Komponente (B) nach Anspruch 16 enthält.

21. Vorrichtung nach Anspruch 20, **dadurch gekennzeichnet, dass** sie drei Abteilungen aufweist, wobei eine Abteilung die Komponente (A) enthält, eine zweite Abteilung die Komponente (B) enthält, wobei mindestens eine der Komponenten (A) und (B) in Form einer wasserfreien Zusammensetzung vorliegt, und eine dritte Komponente ein zum Färben geeignetes, wässriges Medium enthält, wobei das Medium dazu vorgesehen ist, vor der Anwendung mit mindestens einer der Komponenten (A) und (B) vermischt zu werden.
